# EUROPÄISCHE PATENTANMELDUNG

(11) **EP 1 426 033 A2**
(43) Veröffentlichungstag der Anmeldung: **09.06.2004**
(21) Anmeldenummer: 04001875.6
(22) Anmeldetag: 29.01.2004
(51) Int. Cl.: A61K 7/06

(54) **Emulsionsförmiges Haarbehandlungsmittel mit Gehalt an Feststoffpartikeln**

(71) Anmelder: Wella Aktiengesellschaft, 64274 Darmstadt (DE)
(72) Erfinder: Hannich, Manuela, 63329 Egelsbach (DE); Stein, Bernd, 63768 Hösbach (DE); Franzke, Michael, Dr., 64380 Rossdorf (DE); Doepner-Reichenbach, Ute, 61462 Königstein (DE); Siefert, Julia, 64319 Pfungstadt (DE); Haselbauer, Iris, 64589 Stockstadt (DE)

(57) **Zusammenfassung**

Die Erfindung betrifft ein emulsionsförmiges Haarbehandlungsmittel mit einem Gehalt an (A) mindestens 50 Gew.% Wasser, (B) 5 bis 10 Gew.% mindestens eines hydrophoben Stoffes, vorzugsweise Fettalkohole, Pflanzenöle, flüssige Kohlenwasserstoffe und Silikonöle, (C) mindestens 15 Gew.% eines Emulgators, (D) haarfestigendem Polymer und (E) ungelöstem, partikelförmigem Feststoff, vorzugsweise Silica sowie ein Verfahren zum Erstellen einer Frisur unter Verwendung des erfindungsgemäßen Haarbehandlungsmittels.

## Beschreibung

Gegenstand der Erfindung ist ein emulsionsförmiges Haarbehandlungsmittel mit einem Gehalt an Wasser, hydrophoben Stoffen, Emulgator, haarfestigendem Polymer und in dem Mittel ungelösten, partikelförmigen Feststoffen. Die Erfindung betrifft auch Verfahren zum Erstellen einer Frisur unter Verwendung des erfindungsgemäßen Haarbehandlungsmittels.

Eine permanente Rasta-Frisur ist eine Haartracht aus eng geflochtenen langen Zöpfen aus in der Regel dünnen und extrem stark verfilzten Haarsträhnen. Derartige Frisuren werden auch als Dreadlocks bezeichnet. Ethnische Haare (z.B. afrikanisches oder afro-amerikanisches Haar, sogenanntes kinky hair) bietet aufgrund der natürlichen Beschaffenheit und der natürlichen Krause ideale Voraussetzungen zum Erstellen von Dreadlocks bzw. Rasta-Frisuren. Mit geeigneten Techniken sind Rasta-Frisuren aber auch bei anderen, u.a. auch glatten Haarqualitäten, z.B. mitteleuropäischem oder asiatischem Haar zu verwirklichen. Hierfür ist es aber erforderlich, vor der Frisurenerstellung eine zur starken Verfilzung neigende Haarstruktur zu erzeugen. Dies erfolgt üblicherweise durch eine langwierige Kombination von chemischer Behandlung des Haares mit einem Reduktionsmittel und mechanischer Behandlung (bis zu mehrere Stunden dauerndes Toupieren). Hierbei kommt es zu einer intensiven, irreversiblen Verfilzung der Haare und das Haar wird irreparabel geschädigt. Die mit den herkömmlichen Techniken erstellten permanenten Rasta-Frisuren sind nicht ohne weiteres wieder entfernbar. Für modebewußte Anwender, die eine Rasta-Frisur nur vorübergehend oder nur zu einem bestimmten Anlaß, z.B. für eine Party am Wochenende tragen möchten, am nächsten Tag aber ihre ursprüngliche Frisur ohne Schädigung der Haarstruktur zurück haben möchten, besteht daher ein starkes Bedürfnis nach einer Technik zur Erstellung einer wieder entfernbaren, temporären Rasta- oder Rastaähnlichen Frisur ohne haarschädigende Behandlungsschritte und in möglichst kurzer Zeit, das heißt ohne langwieriges Toupieren. Eine einfachere Variante des bekannten Rast-Looks sind sogenannte Ropes. Dabei handelt es sich um kleine, gedrehte Haarsträhnen.

Die Aufgabe bestand darin, ein Haarbehandlungsmittel zur Verfügung zu stellen, mit welchem es besonders vorteilhaft möglich ist, eine derartige Ropes-Frisur zu erstellen. Einerseits sollte eine ausreichende Haltbarkeit der Frisur von ca. einem Tag, insbesondere 1 bis 3 Tagen gegeben sein, andererseits sollte die Frisur bei Bedarf möglichst unaufwändig wieder entfernbar sein. Das Haarbehandlungsmittel sollte gut partienweise auf Haarsträhnen verteilbar sein und einen Zusammenhalt der Ropes bewirken ohne so klebrig zu sein, dass die Ropes selber aneinander haften oder sich das Haar unangenehm klebrig anfühlt. Die erstellte Frisur sollte dabei elastisch bleiben.

Gegenstand der Erfindung ist ein Haarbehandlungsmittel, welches in Form einer Emulsion aus einer wasserhaltigen, hydrophilen Phase und einer hydrophoben Phase vorliegt und einen Gehalt aufweist an
(A) mindestens 50 Gew.%, bezogen auf die Gesamtzusammensetzung, Wasser
(B) 5 bis 10 Gew.%, vorzugsweise 5 bis 10 Gew.%, bezogen auf die Gesamtzusammensetzung, mindestens eines hydrophoben Stoffes,
(C) mindestens 15 Gew.%, bezogen auf die Gesamtzusammensetzung, mindestens eines Emulgators,
(D) mindestens einem haarfestigenden Polymer,
(E) mindestens einem in dem Haarbehandlungsmittel ungelösten, partikelförmigen Feststoff.

Erfindungsgemäße Haarbehandlungsmittel zeichnen sich durch ein besonderes Klebrigkeitsprofil aus. Es wird eine genügende Haftung der Einzelhaare aneinander erzeugt, was eine ausreichende Stabilität der Ropes bewirkt. Nach dem Trocknen weisen die Haare einen angenehmen, nicht klebrigen Griff auf. Weitere Vorteile des erfindungsgemäßen Produktes ist eine problemlose Auswaschbarkeit, vorzugsweise nur mit Wasser ohne aufwändige mechanische Behandlung.

Unter emulsionsförmiger Konsistenz werden Wasser-in-Öl-, Öl-in-Wasser- und Mikroemulsionen verstanden. Das erfindungsgemäße Mittel ist vorzugsweise eine Wasser-in-Öl-Emulsion.

Das Lösungsmittel der hydrophilen Phase kann aus Wasser oder aus einem Gemisch aus Wasser und mindestens einem Alkohol gebildet sein. Geeignete Alkohole sind insbesondere ein- oder mehrwertige C1- bis C5-Alkohole wie z.B. Ethanol, n-Propanol, Isopropanol, Butanole, Pentanole, Ethylenglykol, Propylenglykole, Butylenglykole, Glycerin oder Pentandiole. Bevorzugte einwertige Alkohole sind Ethanol und Isopropanol.

Bevorzugte mehrwertige Alkohole sind die Propylenglykole und Glycerin. Wasser ist vorzugsweise in einer Menge von 50 bis 65 Gew.% enthalten. Alkohole sind vorzugsweise in einer Menge von 0,1 bis 15 Gew.%, besonders bevorzugt von 1 bis 10 Gew.% enthalten.

### Hydrophober Stoff (B)

Der hydrophobe Stoff ist vorzugsweise in einer Menge von 5 bis 10 Gew.% enthalten. Es kann sich um bei Raumtemperatur (25°C) flüssige oder feste Stoffe handeln. Geeignete Stoffe sind u.a. Wachse oder wachsartige Stoffe, z.B. natürliche, nachwachsende Wachse (Insekten-, Tier- und Pflanzenwachse), fossile Wachse (Erdöl-, Braunkohlen-, Torfwachse oder Ozokerite), synthetische Wachse (Fischer-Tropsch-, Polyethylenoder Amidwachse), höherschmelzende Paraffine, Ester, Fette, langkettige Carbonsäuren oder langkettige (C10- bis C22-) Alkohole, jeweils mit Schmelz- bzw. Erstarrungspunkten oberhalb Raumtemperatur (20°C). Weitere hydrophobe Stoffe sind insbesondere Öle oder ölartige Stoffe, z.B. natürlich vorkommende, nachwachsende Öle (pflanzliche und tierische fette Öle), synthetische Öle, Silikonöle, Mineralöle, etherische Öle, wasserunlösliche, verzweigte oder lineare aliphatische Kohlenwasserstoffe, lineare oder verzweigte Alkohole, insbesondere flüssige Fettalkohole sowie langkettige Ether oder Ester, wobei die genannten Substanzen vorzugsweise jeweils mindestens 8 C-Atome, besondes bevorzugt 8 bis 22 C-Atome aufweisen. Geeignete Kohlenwasserstoffe sind z.B. flüssige Paraffine, Squalan oder Squalene. Weiterhin geeignet sind Ester von drei- und mehrwertigen Alkoholen, insbesondere pflanzlichen Triglyceriden wie z.B. Olivenöl, Mandelöl, Erdnußöl, Sonnenblumenöl sowie synthetische Triglyceride wie z.B. C8-C10-Trifettsäureglycerinester oder auch Jojobaöl. Weiterhin geeignet sind Mono- oder Diester der Formeln R¹-COOR², R¹-COO-R³-OOCR¹ und R²OOC-R³-COOR², wobei R¹ für eine C8- bis C22-Alkylgruppe, R² für eine C3- bis C22-Alkylgruppe und R³ für eine C2- bis C16-Alkylengruppe steht. Geeignet sind auch verzweigte primäre Alkohole, wie sie unter der Bezeichnung Guerbetalkohole bekannt sind.
Als hydrophobe Substanzen sind ausserdem Stoffe geeignet, die üblicherweise als Trübungsmittel in kosmetischen Mitteln eingesetzt werden, insbesondere solche der Formel R¹-COO-(CHR⁴CHR⁵O)ₙ-COR⁶, wobei R¹ für eine C8- bis C22-Alkylgruppe, R⁴ und R⁵ für Wasserstoff oder Methyl und R⁶ für Wasserstoff oder für R¹ steht und n eine Zahl zwischen 1 und 12, vorzugsweise 1, 2, 3 oder 4 bedeutet. Bevorzugt sind Glykoldifettsäureester und Polyethylenglykoldifettsäureester, welche bei Raumtemperatur in fester Form vorliegen.

Der hydrophobe Stoff kann auch eine wassunlösliche Silikonverbindung, insbesondere ein Slikonöl sein, z.B. flüssige cyclische oder lineare Silikone (Dimethylpolysiloxane), flüssige, leicht-flüchtige Silikone, cyclische Dimethylsiloxane mit 3 bis 8, vorzugsweise 4 bis 6 Si-Atomen, insbesondere Cyclotetradimethylsiloxan, Cyclopentadimethylsiloxan oder Cyclohexadimethylsiloxan. Weitere Silikone sind Dimethylsiloxan/Methylalkylsiloxan Cyclocopolymere, z.B. Silicone FZ 3109 von Union Carbide, welches ein Dimethylsiloxan/Methyloctylsiloxan Cyclocopolymer ist. Geeignete Silikonöle sind insbesondere Polydimethylsiloxane, phenylierte Silikone, Polyphenylmethylsiloxane, Phenyltrimethicone, Poly(C1-C20)-alkylsiloxane, Alkylmethylsiloxane.

Besonders bevorzugte hydrophobe Stoffe sind ausgewählt aus Fettalkoholen, Pflanzenölen, bei Raumtemperatur flüssigen Kohlenwasserstoffen und Silikonölen.

### Emulgator (C)

Der Gehalt an Emulgatoren (C) ist vorzugsweise 15 bis 30 Gew.%. Die Emulgatoren können nichtionisch, anionisch, kationisch, amphoter oder zwitterionisch sein oder deren Gemische. Geeignete Emulgatoren sind beispielsweise die im "International Cosmetic Ingredient Dictionary and Handbook", 7. Auflage, Band 2 im Abschnitt "Surfactants", insbesondere im Unterabschnitt "Surfactants - Emulsifying Agents" aufgeführten Emulgatoren. Nichtionische Emulgatoren sind z.B. oxethylierte Fettalkohole, oxethylierte Nonylphenole, Fettsäuremonound -diglyceride, ethoxyliertes und hydriertes oder nicht hydriertes Rizinusöl, Fettsäurealkanolamide, oxethylierte Fettsäureester. Kationische Emulgatoren sind z.B. langkettige quaternäre Ammoniumverbindungen wie sie unter den CTFA-Bezeichnungen "Quaternium" bekannt sind wie z.B. Alkyltrimethylammoniumsalze oder Dialkyldimethylammoniumsalze mit C8- bis C22-Alkylgruppen. Anionische Emulgatoren sind z.B. Fettalkoholsulfate, Alkylethersulfate, Alkylbenzolsulfonate. Amphotere Emulgatoren sind z.B. Betaine wie Fettsäureamidoalkylbetaine und Sulfobetaine und C8- bis C22-Alkylbetaine.

Bevorzugte Emulgatoren sind z.B.
- Ethoxylierte Fettalkohole, Fettsäuren, Fettsäureglyceride oder Alkylphenole, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin
- Anlagerungsprodukte von 5 bis 60 mol, vorzugsweise 20 bis 50 mol Ethylenoxid an Rizinusöl oder an gehärtetes (hydriertes) Rizinusöl.
- Mono-, Di- und/oder Triester der Phosphorsäure mit Anlagerungsprodukten von 2 bis 30 mol Ethylenoxid an C8-bis C22-Fettalkohole
- Fettsäurezuckerester, insbesondere Ester aus Saccharose und ein oder zwei C8- bis C22-Fettsäuren, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Polyglycerylfettsäureester, insbesondere aus ein, zwei oder mehreren C8- bis C22-Fettsäuren und Polyglycerin mit vorzugsweise 2 bis 20 Glyceryleinheiten.

Eine bevorzugte Emulgatorenmischung besteht aus nichtionischen Emulgatoren, wobei ein, zwei oder mehr Emulgatoren ausgewählt sind aus ethoxylierten und hydriertem Rizinusöl und mindestens ein weiterer Emulgator ausgewählt ist aus ethoxylierten Fettalkoholen.

### Haarfestigende Polymere (D)

Der Gehalt an haarfestigenden Polymeren (D) ist vorzugsweise 0,1 bis 15 Gew.%, insbesondere 0,5 bis 10 Gew.%. Die Polymere können nichtionisch, anionisch, kationisch, zwitterionisch oder amphoter sein oder deren Mischungen. Es kann ein synthetisches oder ein natürliches Polymer sein. Unter natürlichen Polymeren werden auch chemisch modifizierte Polymere natürlichen Ursprungs verstanden. Unter filmbildenden Polymeren werden erfindungsgemäß solche Polymere verstanden, die bei Anwendung in 0,01 bis 5%iger wässriger, alkoholischer oder wässrig-alkoholischer Lösung in der Lage sind, auf dem menschlichen Haar einen Polymerfilm abzuscheiden.

Geeignete synthetische, nichtionische filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkylund Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide; Polyvinylalkohole, sowie Polyethylenglykol/Polypropylenglykol Copolymere. Besonders bevorzugt sind Polyvinylpyrrolidon und Polyvinylpyrrolidon/Vinylacetat Copolymere.

Geeignete anionische filmbildende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin, Monoethanolamin oder Tetrahydroxypropylethylendiamin und Ammoniak, NaOH und andere. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind zum Beispiel Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere, sind Polymere, welche neben sauren oder aniionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester, wie sie z.B. unter dem Handelsnamen Amphomer® oder Amphomer® LV-71 der Firma NATIONAL STARCH, USA erhältlich sind. Weitere Beispiele für haarfestigende Copolymere mit Säuregruppen sind Copolymere von Acrylsäure, Methylacrylat und Methacrylamidopropyltrimethylammoniumchlorid (INCI-Bezeichnung: Polyquaternium-47), wie sie beispielswiese unter der Handelsbezeichnung Merquat® 2001 vertrieben werden, Copolymere aus Acrylamidopropyltrimethylammoniumchlorid und Acrylaten, oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43). Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer. Geeignet sind auch die zwitterionischen amphoteren Polymere, die in der JP 10-29919 oder in der JP 10-25344 beschrieben sind.

Geeignete kationische Polymere sind Polymere mit kationischen oder basischen, d.h. kationisierbaren Gruppen. Diese Polymere enthalten stickstoffhaltige Gruppen wie z.B. primäre, sekundäre oder tertiäre Amine. Die basische Gruppe ist entweder in der Polymerkette oder vorzugsweise als Substituent an einem oder mehreren Monomeren enthalten. Das Polymer mit basischen Gruppen kann ein natürliches oder ein synthetisches Homo- oder Copolymer mit aminsubstituierten Monomereinheiten sowie gegebenenfalls mit nicht-basischen Comonomeren sein. Geeignete Polymere mit basischen Gruppen sind z.B. Copolymere von aminsubstituierten Vinylmonomeren und nicht aminsubstituierten Monomeren. Aminsubstituierte Vinylmonomere sind z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise niedere Alkylgruppen wie z.B. C1- bis C7-Alkylgruppen, besonders bevorzugt C1- bis C3-Alkylgruppen sind.

Nicht aminsubstituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Maleinsäureanhydrid, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit kationischen Gruppen enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren Monomeren enthalten sind. Die Ammoniumgruppen enthaltenden Monomere können mit den oben genannten nicht aminsubstituierten Monomeren copolymerisiert sein. Geeignete ammoniumsubstituierte Vinylmonomere sind z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16), quaternisiertes Vinylpyrrolidon/Dimethylaminoethylmethacrylat Copolymer (Polyquaternium-11), Homo- und Copolymere von Dimethyldiallylammoniumchlorid (Polyquaternium-6 und -7), quaternisierte Hydroxyethylcellulose (Polyquaternium-10), quaternisierte Guarderivate oder Polymere aus Vinylpyrrolidon, Dimethylaminopropylmethacrylamid und Methacryloylaminopropyllauryldimethylammoniumchlorid (Polyquaternium-55, Styleze® W-20).

Von den kationischen filmbildenden Polymeren, die in dem erfindungsgemäßen Mittel enhalten sein können, ist z.B. Polyvinylpyrrolidon/Dimethylaminoethylmethacrylat Copolymer geeignet. Weitere kationische Polymere sind beispielsweise das Copolymer aus Polyvinylpyrrolidon und Imidazoliminmethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, quaternierte Ammoniumsalze aus Hydroxyethylcellulose und einem trimethylammonium-substituierten Epoxid und Vinylpyrrolidon/Methacrylamidopropyltrimethylammoniumchlorid Copolymere.

### Partikelförmige Feststoffe

Der Gehalt an partikelförmigen Feststoffen (E) ist vorzugsweise 0,1 bis 5, insbesondere 0,5 bis 2 Gew.%. Die Partikel können verschiedene Formen und Dichten aufweisen, sie können sphärisch, oval oder irregulär sein. Die mittlere Partikelgröße kann z.B. von 1 nm bis 100 Mikrometer betragen. Die Partikelgröße, d.h. der maximale Abstand zwischen zwei sich gegenüberliegenden Punkten eines Partikels, kann z.B. mit einem Lasergranulometer bestimmt werden.

Partikelförmige Feststoffe sind z.B. solche auf Basis von Talk, Kaolin (hydratisiertes Aluminiumsilikat), gefällten Carbonaten, gefällten Hydrogencarbonaten, Hydroxyapatit, Silica, Silikaten, Aluminaten, Tonerden, Mica, Salzen, insbesondere anorganische Metallsalze, Metalloxiden z.B. Titandioxid,Zinkoxod, Aluminiumoxid, Zirkoniujmoxid, Ceroxid, Minerale und unlösliche, nicht auf dem Haar filmbildende partikelförmige Polymere, z.B. in Form von sphärischen Mikropartikeln (Microspheres) oder nicht-sphärischen Mikropartikeln, Polymerpulvern, expandierten Mikrosphären, Silikonmikrosphären, Polysaccharidmikrosphären.

Partikelförmige Feststoffe sind insbesondere Nylonpulver, Polyethylenpulver, Poly-beta-alaninpulver, Polyperfluoralkylenpulver, Acrylcopolymerpulver, Acrylatpolymerpulver, Polyamidpulver, Polystyrolpulver, Polyesterpulver, Polyurethanpulver, expandierte Mikrosphären aus thermoplastischem Material, z.B. Expancel 551 DE, Silkonharzmikropartikel, z.B. Tospearl. Besonders bevorzugt ist Silica, insbesondere mit einer mittleren Teilchengröße von 1 bis 100 nm oder von 4 bis 20 nm sowie Perlglanzpigmente mit einer mittleren Teilchengröße von 1 bis 100 Mikrometer oder von 10 bis 60 Mikrometer.

Weitere, bevorzugte Partikel sind Pigmente, bzw. farbige Partikel, z.B. ausgewählt aus einem oder mehreren organischen Pigmenten, anorganisch-organischen Mischpigmenten, anorganischen Pigmenten natürlichen Ursprungs, Weißpigmenten, Schwarzpigmenten, Fluoreszenzpigmenten, Phosphoreszenzpigmenten, farbigen Metalloxiden, farbigen Metallhydroxiden, farbigen Metalloxidhydraten, farbigen Mischphasenpigmenten, farbigen schwefelhaltigen Silicaten, farbigen Metallsulfiden, farbigen komplexen Metallcyaniden, farbigen Metallsulfaten, farbigen Metallchromaten, farbigen Metallmolybdaten, Bronzepigmenten, schwarzem Eisenoxid, gelbem Eisenoxid, rotem Eisenoxid, braunem Eisenoxid, Manganviolett, Ultramarin, Chromoxidhydrat, Eisenblau, Carmine und Pigmenten auf Mica- oder Glimmerbasis, welche mit einem Metalloxid oder einem Metalloxychlorid sowie mit einem farbgebenden Stoff, ausgewählt aus Eisenoxiden, Eisenblau, Ultramarin, Carmin und Chromoxid beschichtet sind.

In einer besonderen Ausführungsform handelt es sich bei dem partikelförmigen Feststoff um ein Pigment auf Basis von Glas, z.B. auf Basis von Borsilikatglas, vorzugsweise beschichtet mit Metalloxiden wie z.B. Eisenoxid, Titandioxid und/oder Zinnoxid. Borsilikatglas ist z.B. Calcium-Natrium-Borsilikatglas. Pigmente auf Glasbasis sind z.B. solche mit der INCI-Bezeichnung Calcium Sodium Borosilicate. Handelsprodukte sind z.B. solche mit der Bezeichnung Reflecks® oder Reflecks® Dimensions, z.B. Reflecks® Dimensions Sparkling Blue.

In einer bevorzugten Ausführungsform enthält das erfindungsgemäße Haarbehandlungsmittel
(A) 50 bis 65 Gew.%, bezogen auf die Gesamtzusammensetzung, Wasser
(B) 5 bis 10 Gew.%, bezogen auf die Gesamtzusammensetzung, mindestens eines hydrophoben Stoffes, ausgewählt aus Fettalkoholen, Pflanzenölen, bei Raumtemperatur flüssigen Kohlenwasserstoffen und Silikonölen,
(C) 15 bis 30 Gew.%, bezogen auf die Gesamtzusammensetzung, mindestens eines Emulgators,
(D) 0,1 bis 15 Gew.% mindestens eines haarfestigenden Polymers, ausgewählt aus nichtionischen und kationischen Vinyllactampolymeren, insbesondere Polyvinylpyrrolidon und/oder Polyquaternium-55 und
(E) 0,5 bis 2 Gew.% Silica.

Zusätzlich zu den vorstehend genannten Inhaltsstoffen können die erfindungsgemäßen Produkte weitere, übliche kosmetische Zusatzstoffe enthalten:
- Kosmetische Anfärbestoffe in einer Menge bis zu 6 Gew.%, vorzugsweise 0,1 bis 4 Gew.%, z.B. C.I. Pigment Red 4 (C.I. 12 085), C.I. Pigment Green (C.I. 74 260), und/oder C.I. Vat Blue 4 (C.I. 69 800)
- Parfüm- und Duftstoffe in einer Menge von bis zu 2 Gew.%, vorzugsweise 0,01 bis 1 Gew.%
- Lichtschutz- und Konservierungsmittel in einer Menge von bis zu 1 Gew.%, vorzugsweise 0,01 bis 0,5 Gew.%
- Haarpflegende Zusätze wie z.B. Betain, Panthenol in einer Menge von bis zu 5 Gew.%, vorzugsweise 0,01 bis 4 Gew.%.

### Applikations- und Verpackungsformen

Das erfindungsgemäße Produkt kann je nach Konsistenz in geeigneten Verpackungen wie z.B. Tiegeln, Tuben, Flaschen oder ähnlichen abgefüllt werden. Die Verpackungen können mit einer Pumpvorrichtung, z.B. einem mechanisch betriebenen Pumpspender zur Ausbringung der Produktmasse versehen sein.

Eine besondere Ausführungsform ist ein Produkt zur Haarbehandlung, bei welchem eine erfindunsgemäße Zusammensetzung zusammen mit einem geeigneten Treibmittel in einer druckfesten Verpackung abgefüllt und mit einer Vorrichtung zum Verschäumen (Schaumkopf) versehen ist. Geeignete Treibmittel sind insbesondere verflüssigte Treibgase wie z.B. Propan, n-Butan, Isobutan, fluorierte Kohlenwasserstoffe wie z.B. 1,1-Difluorethan oder 1,1,1,2-Tetrafluorethan oder Dimethylether. Diese Treibgase können einzeln oder im Gemisch eingesetzt werden, z.B. ein Gemisch von Propan und/oder Butan und Dimethylether. Besonders bevorzugt ist ein Gemisch von Propan und Butan. Typische Abfüllverhältnisse liegen im Bereich von ca. 80 bis 98 Gew.% Wirkstoffmischung zu 2 bis 20 Gew.% Treibmittel. Die druckfeste Verpackung kann aus einem beliebigen, für Aerosol Sprüh- oder Schaumprodukte bekanntem Material gefertigt sein. Geeignete Materialien sind insbesondere Metalle wie Aluminium oder Weißblech. Als Schaumköpfe können handelsübliche Schaumköpfe verwendet werden.

### Anwendungsverfahren

Ein weiterer Gegenstand der Erfindung ist ein Verfahren zum Erstellen einer Frisur, wobei ein erfindungsgemäßes Haarbehandlungsmittel auf trockenes oder feuchtes Haar aufgebracht wird und anschließend die gewünschte Frisur erstellt wird. Das Produkt kann gezielt auf einzelnen, abgeteilten Haarsträhnen verteilt werden, wobei das Produkt vorzugsweise vom Haaransatz zu den Spitzen in die abgeteilte Haarsträhne eingearbeitet wird. Die Einsatzmenge ist je nach Haarlänge und gewünschtem Effekt eine erbsen- bis haselnussgroße Menge. Während der Zeit, in der das Produkt auf dem Haar noch nicht vollständig getrocknet ist, lassen sich die Strähnen leicht in die gewünschte Form bringen, z.B. können durch einfaches Verdrehen um sich selbst, beginnend am Haaransatz, Ropes geformt werden. Nach vollständigem Trocknen bleiben die Ropes stabil erhalten, wobei sich die Haare angenehm anfühlen ohne klebrig zu sein. Die Ropes können am darauffolgenden Tag nochmals mit dem erfindungsgemäßen Mittel behandelt werden, um sie haltbarer zu machen. Die Ropes können durch einfaches Waschen der Haare leicht wieder entfernt werden. Die besonderen Vorteile sind eine kurze Erstellungszeit sowie eine unproblematische, leichte Wiederentfernbarkeit.

Die nachfolgenden Beispiele sollen den Gegenstand der Erfindung näher erläutern.

### Beispiele

| Beispiel 1 - Flüssig-Styler | |
|---|---|
| 50 - 65 g | Wasser |
| 20 g | Emulgator |
| 15 g | haarfestigende Polymere, z.B. Mischung aus Polyquaternium-55 und Polyvinylpyrrolidon |
| 5 g | mehrwertiger Alkohol, z.B. Propylenglykol |
| 5 - 10 g | hydrophobe Phase, z.B. Fettalkohole |
| 0,5 - 2 g | Silica |

| Beispiel 2 - Flüssig-Styler | |
|---|---|
| 3,4 g | PEG-25 Hydrogenated Castor Oil |
| 3,3 g | PEG-40 Hydrogenated Castor Oil |
| 3,3 g | Ceteareth-25 |
| 5 g | Polyquaternium-55 |
| 10 g | Polyvinylpyrrolidon |
| 5 g | mehrwertiger Alkohol, z.B. Propylenglykol |
| 7,50 g | Cetylalkohol |
| 1 g | Silica |
| ad 100 g | Wasser |

| Beispiel 3 - Aerosolschaum | |
|---|---|
| 50 g | Wasser |
| 25 g | Emulgator |
| 10 - 15 g | haarfestigende Polymere, z.B. Mischung aus Polyquaternium-55 und Polyvinylpyrrolidon |
| 3 - 6 g | mehrwertiger Alkohol, z.B. Propylenglykol |
| 5 - 10 g | hydrophobe Phase, z.B. Fettalkohole |
| 0,5 - 2 g | Silica |
| 10 g | Treibmittel, z.B. Propan, n-Butan |

| Beispiel 4 - Aerosolschaum | |
|---|---|
| 50 g | Wasser |
| 25 g | Emulgator |
| 6 g | Polyquaternium-55 |
| 6 g | Polyvinylpyrrolidon |
| 4,5 g | Propylenglykol |
| 7,5 g | Cetylalkohol |
| 1 g | Silica |
| 10 g | Propan/n-Butan |

| Beispiel 5 - Farbeffekt Flüssig-Styler | |
|---|---|
| 3,4 g | PEG-25 Hydrogenated Castor Oil |
| 3,3 g | PEG-40 Hydrogenated Castor Oil |
| 3,3 g | Ceteareth-25 |
| 5 g | Polyquaternium-55 |
| 10 g | Polyvinylpyrrolidon |
| 5 g | mehrwertiger Alkohol, z.B. Propylenglykol |
| 7,50 g | Cetylalkohol |
| 1 g | Reflecks® Dimensions Sparkling Blue (INCI: Calcium Sodium Borosilicate (and) Titanium Dioxide (and) Tin Oxide) |
| ad 100 g | Wasser |

## Patentansprüche

1. Haarbehandlungsmittel, welches in Form einer Emulsion aus einer wasserhaltigen, hydrophilen Phase und einer hydrophoben Phase vorliegt und einen Gehalt aufweist an
(A) mindestens 50 Gew.%, bezogen auf die Gesamtzusammensetzung, Wasser
(B) 5 bis 10 Gew.%, bezogen auf die Gesamtzusammensetzung, mindestens eines hydrophoben Stoffes,
(C) mindestens 15 Gew.%, bezogen auf die Gesamtzusammensetzung, mindestens eines Emulgators,
(D) mindestens einem haarfestigenden Polymer,
(E) mindestens einem in dem Haarbehandlungsmittel ungelösten, partikelförmigen Feststoff.

2. Haarbehandlungsmittel nach Anspruch 1, **dadurch gekennzeichnet, dass** das Wasser (A) in einer Menge von 50 bis 65 Gew.%, der hydrophobe Stoff (B) in einer Menge von 5 bis 10 Gew.%, der Emulgator (C) in einer Menge von 15 bis 30 Gew.%, das Polymer (D) in einer Menge von 0,1 bis 15 Gew.% und der partikelförmige Feststoff (E) in einer Menge von 0,5 bis 2 Gew.% enthalten sind.

3. Haarbehandlungsmittel nach einem der vorhergehenden Ansprüche, **dadurch gekennzeichnet, dass** die hydrophile Phase ein Gemisch ist aus Wasser und ein- oder mehrwertigen C1- bis C6-Alkoholen.

4. Haarbehandlungsmittel nach einem der vorhergehenden Ansprüche, **dadurch gekennzeichnet, dass** der Emulgator (C) ausgewählt ist aus
a) Anlagerungsprodukten von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
b) C12- bis C22-Fettsäuremono- und -diestern von Anlagerungsprodukten von 1 bis 30 mol Ethylenoxid an Glycerin,
c) Anlagerungsprodukten von 5 bis 60 mol Ethylenoxid an Rizinusöl oder an gehärtetes Rizinusöl,
d) Mono-, Di- und/oder Triestern der Phosphorsäure mit Anlagerungsprodukten von 2 bis 30 mol Ethylenoxid an C8- bis C22-Fettalkohole
oder aus Gemischen dieser Emulgatoren.

5. Haarbehandlungsmittel nach einem der vorhergehenden Ansprüche, **dadurch gekennzeichnet, dass** der hydrophobe Stoff (B) ausgewählt ist aus Fettalkoholen, Pflanzenölen, bei Raumtemperatur flüssigen Kohlenwasserstoffen und Silikonölen.

6. Haarbehandlungsmittel nach einem der vorhergehenden Ansprüche, **dadurch gekennzeichnet, dass** das haarfestigende Polymer (D) ausgewählt ist aus
- nichtionischen Polymeren, welche aus mindestens einem der folgenden Monomere aufgebaut sind:
Vinyllactam, Vinylester, Vinylalkohol, Acrylamid, Methacrylamid, Alkylacrylamid, Dialkylacrylamid, Alkylmethacrylamid, Dialkylmethacrylamid, Alkylacrylat, Alkylmethacrylat, Propylenglykol oder Ethylenglykol, wobei die Alkylgruppen dieser Monomere C1- bis C7-Alkylgruppen sind;
- anionischen Polymeren, welche aus mindestens einem der folgenden Monomere aufgebaut sind:
Acrylsäure, Methacrylsäure, Crotonsäure, Maleinsäure bzw. Maleinsäureanhydrid oder deren Monoester, Aldehydocarbonsäuren oder Ketocarbonsäuren;
- kationischen Polymeren, welche aus mindestens einem Monomer, das mindestens eine kationische oder kationisierbare Gruppe enthält, aufgebaut sind;
- amphoteren Polymeren, welche entweder aus mindestens einem Monomer, das sowohl mindestens eine Säuregruppe als auch mindestens eine kationische oder kationisierbare Gruppe enthält, aufgebaut sind oder welche aus mindesten einem ersten Monomer, das mindestens eine Säuregruppe entält und mindestens einem zweiten Monomer, das mindestens eine kationische oder kationisierbare Gruppe enthält, aufgebaut sind.

7. Haarbehandlungsmittel nach einem der vorhergehenden Ansprüche, **dadurch gekennzeichnet, dass** der partikelförmige Feststoff (E) ausgewählt ist aus partikelförmigen Stoffen auf Basis von Talk, Polymerpulvern, expandierten Mikrosphären, Silikonmikrosphären, gefällten Carbonaten, gefällten Hydrogencarbonaten, Hydroxyapatit, Silica, Silikaten, Aluminaten, Tonerden, Mica, Salzen, Metalloxiden, Polysaccharidmikrosphären, Pigmenten auf Basis von mit Metalloxiden beschichtetem Borsilikatglas.

8. Haarbehandlungsmittel nach einem der vorhergehenden Ansprüche, **dadurch gekennzeichnet, dass** es einen Gehalt aufweist an
(A) 50 bis 65 Gew.%, bezogen auf die Gesamtzusammensetzung, Wasser
(B) 5 bis 10 Gew.%, bezogen auf die Gesamtzusammensetzung, mindestens eines hydrophoben Stoffes, ausgewählt aus Fettalkoholen, Pflanzenölen, bei Raumtemperatur flüssigen Kohlenwasserstoffen und Silikonölen,
(C) 15 bis 30 Gew.%, bezogen auf die Gesamtzusammensetzung, mindestens eines Emulgators,
(D) 0,1 bis 15 Gew.% mindestens eines haarfestigenden Polymers, ausgewählt aus nichtionischen und kationischen Vinyllactampolymeren und
(E) 0,5 bis 2 Gew.% Silica.

9. Haarbehandlungsmittel nach einem der vorhergehenden Ansprüche, **dadurch gekennzeichnet, dass** es zusätzlich ein Treibmittel enthält.

10. Verfahren zum Erstellen einer Frisur, wobei ein Haarbehandlungsmittel nach einem der vorhergehenden Ansprüche auf trockenes oder feuchtes Haar aufgebracht wird und anschließend die gewünschte Frisur erstellt wird.
